# EUROPÄISCHE PATENTSCHRIFT

(11) **EP 1 519 689 B1**
(45) Veröffentlichungstag und Bekanntmachung des Hinweises auf die Patenterteilung: **04.09.2013**
(21) Anmeldenummer: 03725091.7
(22) Anmeldetag: 25.04.2003
(51) Int. Cl.: A61B 17/28, A61B 17/29

(54) **Medizinisches Instrument mit Griffanordnung**
Medical instrument with gripping arrangement
Instrument médical avec dispositif de préhension

(30) Priorität: 10.05.2002 DE 10222042
(43) Veröffentlichungstag der Anmeldung: 06.04.2005
(73) Patentinhaber: Karl Storz GmbH & Co., 78532 Tuttlingen (DE)
(72) Erfinder: SZABO, Zoltan, San Francisco, CA 94114 (US)
(74) Vertreter: Heuckeroth, Volker
(86) Internationale Anmeldenummer: PCT/EP2003/004289
(87) Internationale Veröffentlichungsnummer: WO 2003/094755

(56) Entgegenhaltungen:
- DE-A- 4 211 417
- DE-A- 19 860 444
- US-A- 1 116 099
- US-A- 3 894 336
- US-A- 4 961 742
- US-A- 5 690 636

## Beschreibung

Die Erfindung betrifft ein medizinisches Instrument, mit einer Handhabe, die zwei im wesentlichen stabförmige Griffteile aufweist, die jeweils eine Grifffläche aufweisen.

Aus dem DE-Firmenprospekt der Karl Storz GmbH & Co. KG, STORZ - Karl Storz Endoskope, Band "Endoskopische Chirurgie", 2. Ausgabe 1/94, Seite NH 3 A ist ein medizinisches Instrument in Form eines Nadelhalters bekannt, dessen Handhabe zwei stabförmige Griffteile aufweist, die sehr schlank bauend sind, d.h. einen im Verhältnis zu ihrer Länge kleinen Querschnitt aufweisen. Entsprechend ist die an jedem Griffteil ausgebildete, als Grifffläche dienende Umfangsfläche der Griffteile klein. Die Griffteile dieses bekannten Instruments sind daher möglicherweise als nicht optimal ergonomisch anzusehen, weil die Gesamtgrifffläche der Griffteile dieses Instruments im Verhältnis zur Handinnenfläche der das Instrument bedienenden Person klein ist.

Die Handhabe eines derartigen Instruments hat nicht nur die Aufgabe, das Instrument in der Hand zu halten, sondern die Griffteile eines solchen Instruments sind üblicherweise relativ zueinander beweglich ausgeführt, um durch Betätigen der Griffteile ein Werkzeug, beispielsweise Maulteile, am distalen Ende des Instruments zu betätigen. Das Betätigen des Werkzeugs am distalen Ende des Instruments mittels der Griffteile am proximalen Ende erfordert in der Regel ein sicheres Handgefühl, das wiederum von den ergonomischen Eigenschaften der Griffteile beeinflußt wird. Somit besteht ein Bedürfnis, die Ergonomie des zuvor genannten Instruments zu verbessern, insbesondere an die jeweiligen Bedürfnisse und Voraussetzungen des bedienenden Arztes anzupassen.

Daneben sind medizinische Instrumente bekannt, deren Handhabe bzw. Handgriff als Ganzes vom Schaft des Instruments abnehmbar ist, wie beispielsweise in der DE 198 60 444 A1 beschrieben ist. Es handelt sich dabei um vollwertige Handhaben bzw. Handgriffe, die entsprechend auch mit einer Kraftübertragungsmechanik zum Betätigen des Werkzeugs am distalen Ende des Instruments ausgestattet sind. Mit solchen abnehmbaren Handhaben kann zwar das jeweilige Instrument durch Austauschen des Handgriffs gegen einen anderen an die jeweiligen Bedürfnisse des bedienenden Arztes angepaßt werden, jedoch sind derartige vollwertige bzw. vollständige Handhaben konstruktiv aufwendig und kostspielig, wenn mehrere Sätze derartiger Handhaben unterschiedlicher Größe und Form bereitgehalten werden.

Aus US-A-3 894 336 ist eine Einwegschere zum Schneiden chirurgischer Bandagen, Nähte und dergleichen bekannt, bei der die als Fingerringe ausgebildeten Griffteile von den Scherenarmen abnehmbar sind.

DE 42 11 417 A1 offenbart ein chirurgisches Endoinstrument, bei dem die Griffteile vom Instrument abgenommen werden können.

US-A-1 116 099 offenbart ein chirurgisches Instrument mit abnehmbaren Griffteilen.

Der Erfindung liegt daher die Aufgabe zugrunde, ein medizinisches Instrument bereitzustellen, bei dem mit geringem Kostenaufwand die ergonomischen Eigenschaften des Instruments verbessert, insbesondere an unterschiedliche Bedürfnisse der Bedienungsperson anpassbar sind.

Erfindungsgemäß wird diese Aufgabe durch ein medizinisches Instrument gemäß Anspruch 1 gelöst.

Mit der bei dem erfindungsgemäßen Instrument vorgesehenen Griffanordnung kann ein bereits vorhandenes Instrument ohne Veränderung des Instruments selbst hinsichtlich seiner ergonomischen Handhabungseigenschaften verbessert werden, indem an die Griffteile jeweils eine erfindungsgemäß vorgesehene Griffschale angebracht wird, durch die die Grifffläche der Griffteile vergrößert und/oder formverändert wird. Das Halten bzw. Greifen des medizinischen Instruments wird mittels der Griffschalen, die eine neue Grifffläche definieren, wesentlich verbessert. Darüber hinaus können für ein und dasselbe Instrument eine Mehrzahl von Sätzen derartiger Griffschalen mit verschiedenen Größen und/oder Formen bereitgehalten werden, wodurch die Handhabe des Instruments an die jeweiligen Bedürfnisse der Bedienungsperson angepaßt werden kann. Im Unterschied zu einer abnehmbaren vollständigen Handhabe wie im Stand der Technik hat die erfindungsgemäße Griffanordnung den Vorteil, auf wesentlich kostengünstigere Weise eine Veränderung der Handhabungseigenschaften des Instruments zu ermöglichen.

Die Griffschalen sind vom proximalen Ende der Griffteile auf diese aufschiebbar.

Zusätzlich können die Griffschalen seitlich auf die Griffteile aufsteckbar sein. Durch diese Maßnahmen können die Griffschalen auf vorteilhaft einfach zu handhabende Weise mit dem jeweiligen Griffteil verbunden werden. Die aufschiebbare Ausgestaltung der Griffschalen eignet sich insbesondere auch für solche Griffteile, die im wesentlichen gerade sind, während die aufsteckbare Variante für geschwungene bzw. gekrümmte Griffteile besser geeignet sein kann. Unter "seitlich" aufsteckbar ist zu verstehen, daß die Griffschalen Seite an Seite mit den Griffteilen liegend quer zur Längsrichtung an die Griffteile anbringbar sind.

In einer weiteren bevorzugten Ausgestaltung sind die Griffschalen als Klemmteile zum Anklemmen an die Griffteile ausgebildet. Hierbei ist von Vorteil, daß die Griffschalen auf einfach zu handhabende Weise lediglich an die Griffteile angeklemmt werden müssen und dadurch bereits zumindest teilweise oder sogar vollständig fixiert sind, ohne daß weitere Fixierungsmaßnahmen wie Anziehen von Schrauben oder dgl. vorgenommen werden müssen.

In einer weiteren bevorzugten Ausgestaltung weisen die Griffschalen eine Vertiefung oder Ausnehmung auf, in der die Griffteile in befestigtem Zustand der Griffschalen zumindest teilweise aufgenommen sind.

Hierbei ist von Vorteil, daß sich die Griffschalen an den Griffteilen mit besonders sicherem Halt verbinden lassen, wobei, wie in einer weiteren bevorzugten Ausgestaltung vorgesehen ist, die Vertiefungen bzw. Ausnehmungen auch so ausgebildet werden können, daß die Griffteile im wesentlichen formschlüssig darin aufgenommen werden. Auf diese Weise ist es möglich, die Griffschalen bei entsprechender Formgebung der Vertiefungen oder Ausnehmungen auch verdrehgesichert an den Griffteilen in einer vorbestimmten, durch den Formschluß leicht auffindbaren Drehposition zu verbinden.

In diesem Zusammenhang ist es weiterhin bevorzugt, wenn die Vertiefungen oder Ausnehmungen so ausgebildet sind, daß die Griffteile darin im Querschnitt gesehen vollständig aufgenommen sind.

Hierbei ist von Vorteil, daß die im befestigten Zustand der Griffschalen an den Griffteilen einander zugewandten Seiten der Griffschalen eine gleichmäßige kantenfreie Oberfläche bilden können. Die Vertiefungen oder Ausnehmungen können dann als allseitig geschlossene Bohrungen bzw. Öffnungen ausgebildet sein.

In einer weiteren bevorzugten Ausgestaltung sind im an den Griffteilen befestigten Zustand einander zugewandte Seiten der Griffschalen randseitig jeweils abgerundet ausgebildet.

Diese Maßnahme hat insbesondere dann einen Vorteil, wenn beide Griffteile bis zur Anlage aneinander geschlossen werden können. Durch die randseitige Abrundung der einander zugewandten Seiten der Griffschalen wird nun vorteilhafterweise vermieden, daß beim Zudrücken der Griffteile Haut, vor allem Haut der Handunterkante, zwischen den Griffschalen eingeklemmt wird. Eine andere Möglichkeit, ein derartiges Einklemmen von Haut zu vermeiden, besteht darin, die Griffschalen so auszugestalten, sei es mit einer Vertiefung/Ausnehmung oder ohne, daß die einander zugewandten Seiten der Griffschalen von den einander zugewandten Seiten der Griffteile zurück versetzt sind.

In einer weiteren bevorzugten Ausgestaltung weisen die Griffschalen jeweils zumindest ein Element zum Festlegen der Griffschalen an den Griffteilen in Längsrichtung und/oder Querrichtung zu den Griffteilen und/oder zum Verdrehsichern der Griffschalen um die Längsrichtung der Griffteile auf.

Mittels dieser Maßnahmen kann sicher gewährleistet werden, daß sich die Griffschalen bei der Bedienung des Instruments relativ zu den Griffteilen, an denen sie befestigt sind, nicht verschieben bzw. verdrehen oder von den Griffteilen unerwünscht abheben lassen.

Dabei ist es bevorzugt, wenn das zumindest eine Element eine Schraube oder eine Raste ist.

Mit einer Schraube, beispielsweise in Form einer Madenschraube, die von außen durch die Griffschale eingedreht wird und beispielsweise gegen das entsprechende Griffteil klemmend in Anlage kommt, kann ein besonders sicherer Halt der Griffschalen insbesondere gegen Verschiebung in Längsrichtung bzw. gegen ein Verdrehen um die Längsrichtung an den Griffteilen erreicht werden, während der Vorteil einer Raste als Sicherungselement darin besteht, daß das Anbringen der Griffschalen an den Griffteilen erleichtert ist, weil keine Schraube angezogen werden muß. Mit einer Schraube kann die jeweilige Griffschale auf dem Griffteil auch formschlüssig, beispielsweise durch Einschrauben der Schraube in ein Gewinde- oder Zylinderloch am Griffteil, befestigt werden.

Als weitere bevorzugte Ausgestaltung des zumindest einen Elements zum Festlegen der Griffschalen an den Griffteilen ist das zumindest eine Element eine Lasche, die das entsprechende Griffteil zumindest teilweise umgreift.

Eine solche Lasche sichert die Griffschalen an den Griffteilen vorteilhafterweise besonders wirksam in Querrichtung zu den Griffteilen.

Das zumindest eine Element, um eine Verdrehsicherung der Griffschalen um die Griffteile zu vermeiden, kann vorteilhafterweise auch durch die bereits zuvor genannten Ausnehmungen oder Vertiefungen in den Griffschalen selbst gebildet werden, wenn die Ausnehmungen oder Vertiefungen so ausgebildet sind, daß sie eine im wesentlichen formschlüssige Aufnahme der Griffteile ermöglichen und die Griffteile im Querschnitt nicht rund sind.

In einer weiteren bevorzugten Ausgestaltung weisen die Griffschalen jeweils eine weiche und/oder aufgerauhte Grifffläche auf.

Mit einer weichen Oberfläche werden die ergonomischen Eigenschaften der Griffanordnung vorteilhafterweise weiter verbessert, und mit einer aufgerauhten Oberfläche kann vorteilhafterweise vermieden werden, daß die Griffschalen in der Hand der Bedienungsperson rutschen.

In einer weiteren bevorzugten Ausgestaltung weisen die Griffschalen Verriegelungsmittel zum Verriegeln der Griffteile in verschiedenen relativen Griffstellungen zueinander auf, die vorzugsweise eine stufenlose Verriegelung ermöglichen.

In dieser Ausgestaltung wird mit den erfindungsgemäßen Griffschalen nicht nur eine verbesserte Ergonomie eines medizinischen Instruments erreicht, sondern es kann den Griffteilen eine weitere Funktion gegeben werden, die sie ohne die erfindungsgemäßen Griffschalen vorher ggf. nicht hatten. Diese Funktion besteht darin, die beiden Griffteile in unterschiedlichen Griffstellungen relativ zueinander zu verriegeln. Während bei den bekannten Nadelhaltern die Griffteile bereits mittels eines Rastgesperres in verschiedenen Griffstellungen verriegelbar sind, kann mittels der erfindungsgemäßen Griffschalen nun auch eine stufenlose Verriegelung ermöglicht werden, beispielsweise durch zwei an den Griffschalen vorhandene Elemente, die reibschlüssig zusammenwirken.

Wie bereits weiter oben erwähnt, weist die erfindungsgemäße Griffanordnung vorzugsweise eine Mehrzahl von Sätzen von Griffschalen unterschiedlicher Größen und/oder Formen auf, wodurch ein bereits vorhandenes Instrument an verschiedene Bedürfnisse verschiedener Bedienungspersonen oder auch an verschiedene Bedürfnisse derselben Bedienungsperson für verschiedene Anwendungen angepaßt werden kann.

Die erfindungsgemäß vorgesehenen Griffschalen sind vorzugsweise reinigbar, insbesondere autoklavierbar. Hieraus ergibt sich ein weiterer Vorteil der erfindungsgemäßen Griffanordnung gegenüber solchen Instrumenten, deren Handhabe als Ganzes vom Schaft des Instruments abnehmbar ist. Denn vollwertige abnehmbare Handhaben besitzen aufgrund ihres aufwendigeren Aufbaus zahlreiche Ecken und Nischen, in denen sich Verunreinigungen ansammeln können, die sich nur schwer reinigen lassen. Demgegenüber können die konstruktiv sehr einfach gestalteten Griffschalen einteilig und mit nur wenigen Ecken und Nischen ausgebildet werden, so daß sich Verunreinigungen weniger stark ansammeln oder zumindest leichter entfernen lassen.

Weitere Vorteile und Merkmale ergeben sich aus der nachfolgenden Beschreibung und der beigefügten Zeichnung.

Es versteht sich, daß die vorstehend genannten und nachstehend noch zu erläuternden Merkmale nicht nur in der jeweils angegebenen Kombination, sondern auch in anderen Kombinationen verwendbar sind.

Ausführungsbeispiele der Erfindung sind in den Zeichnungen dargestellt und werden mit Bezug auf diese hiernach näher beschrieben. Es zeigen:
- Fig. 1: eine Seitenansicht eines medizinischen Instruments;
- Fig. 2: das Instrument in Fig. 1 mit einer daran abnehmbar befestigten erfindungsgemäßen Griffanordnung;
- Fig. 3: einen Schnitt entlang der Linie III-III in Fig. 2 in geringfügig vergrößertem Maßstab;
- Fig. 4a) und 4b): ein weiteres Ausführungsbeispiel einer Griffschale einer erfindungsgemäßen Griffanordnung, wobei Fig. 4a) die Griffschale in einer perspektivischen Draufsicht und Fig. 4b) die Griffschale in Fig. 4a) in einer perspektivischen Ansicht von unten zeigt;
- Fig. 5a) und b): Fig. 4a) und b) entsprechende Darstellungen eines weiteres Ausführungsbeispiels einer Griffschale;
- Fig. 6 und 7: zwei Fig. 3 entsprechende Schnittdarstellungen von weiteren Ausführungsbeispielen von Griffanordnungen.

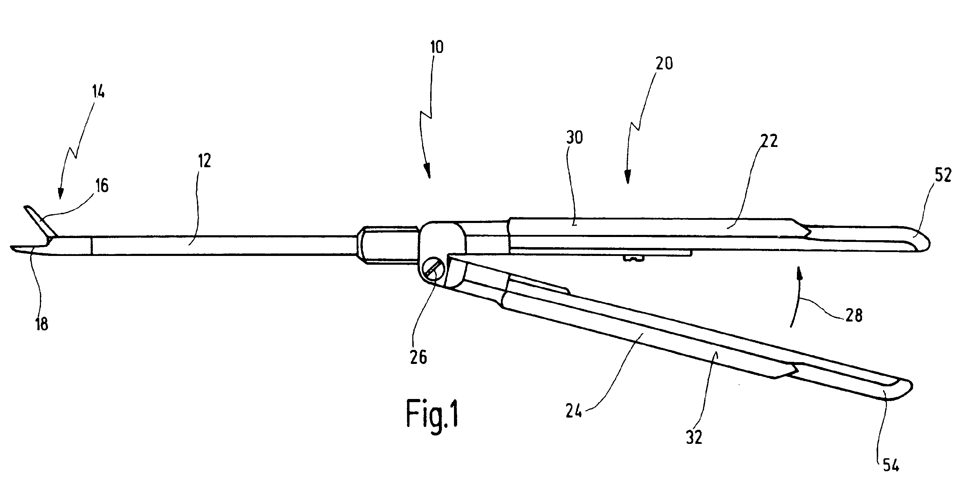

In Fig. 1 bis 3 ist ein mit dem allgemeinen Bezugszeichen 10 versehenes medizinisches Instrument dargestellt. Das Instrument 10 ist in dem Ausführungsbeispiel ein Nadelhalter, kann jedoch im Rahmen der Erfindung auch ein beliebiges anderes medizinisches Instrument, beispielsweise eine Zange zum Schneiden und/oder Fassen oder allgemein zum Präparieren von Gewebe sein.

Das Instrument 10 weist einen Schaft 12 auf, an dessen distalem Ende ein Werkzeug 14 angeordnet ist, das im vorliegenden Ausführungsbeispiel zwei Maulteile 16 und 18 aufweist. Das Maulteil 16 ist beweglich, während das Maulteil 18 unbeweglich ist. Zwischen den Maulteilen 16 und 18 kann beim Schließen der Maulteile 16 und 18 eine nicht dargestellte Nadel gehalten werden.

Am proximalen Ende des Schafts 12 weist das Instrument eine Handhabe 20 auf, die ein erstes Griffteil 22 und ein zweites Griffteil 24 aufweist. Das erste Griffteil 22 ist unbeweglich, während das zweite Griffteil 24 relativ zu dem ersten Griffteil 22 um eine Schwenkachse 26 verschwenkbar ist. Durch Verschwenken des zweiten Griffteils 24 in Richtung eines Pfeiles 28 wird das bewegliche Maulteil 16 gegen das unbewegliche Maulteil 18 geschlossen. Die Kraftübertragung von dem beweglichen Griffteil 24 auf das bewegliche Maulteil 16 erfolgt über eine nicht näher dargestellte Kraftübertragungsmechanik, die eine Zugstange aufweist, wie sie bei derartigen Instrumenten üblich ist.

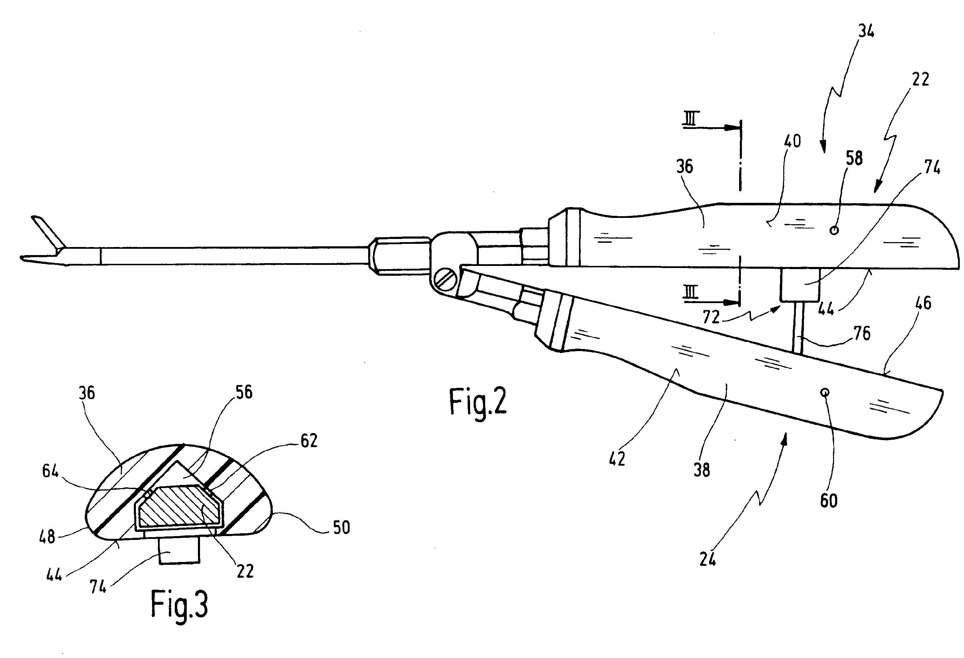

Die Griffteile 22 und 24 sind stabförmig ausgebildet und nehmen im vorliegenden Ausführungsbeispiel einen geraden Verlauf ein. Die Griffteile 22 und 24 sind im Querschnitt gesehen (vgl. Fig. 1 und 3) im Verhältnis zu ihrer Länge schlank bauend ausgebildet. Entsprechend sind Griffflächen 30 und 32 der Griffteile 22 und 24 schmal und nicht optimal an die Handinnenfläche der Hand der Bedienungsperson angepaßt. Die Griffflächen 30 und 32 werden durch die Oberflächen der Griffteile 22 und 24, und zwar der voneinander abgewandten Oberflächen der Griffteile 22 und 24 gebildet.

Um die Griffflächen 30 und 32 der Griffteile 22 und 24 zu vergrößern und ggf. in der Form zu verändern und damit insgesamt ergonomischer zu gestalten, ist gemäß Fig. 2 eine Griffanordnung 34 vorgesehen, die eine erste Griffschale 36 und eine zweite Griffschale 38 aufweist.

Die Griffschalen 36 und 38 sind an dem jeweiligen Griffteil 22 und 24 im Bereich deren Griffflächen 30, 32 abnehmbar befestigt.

Die erste Griffschale 36 weist eine Grifffläche 40 und die zweite Griffschale 38 weist eine zweite Grifffläche 42 auf, die, wie aus einem Vergleich der Fig. 1 und 2 und insbesondere aus Fig. 3 hervorgeht, gegenüber den Griffflächen 30 und 32 der Griffteile 22 und 24 vergrößert ist.

Die Griffschalen 36 und 38 sind hinsichtlich Größe und/oder Form so ausgebildet, daß sie an die jeweiligen Bedürfnisse der Bedienungsperson angepaßt sind.

Im befestigten Zustand der Griffschalen 36 und 38 einander zugewandte Seiten 44 und 46 der Griffschalen 36 und 38 sind randseitig jeweils abgerundet ausgebildet, wie in Fig. 3 für die Griffschale 36 dargestellt ist, die Abrundungen 48 und 50 aufweist. Durch die Abrundungen 48 und 50 wird vermieden, daß Haut der das Instrument 10 haltenden Hand der Bedienungsperson zwischen den Griffschalen 36 und 38 beim Schließen bzw. Zusammendrücken der Griffteile 22 und 24 eingeklemmt wird.

Die Griffschalen 36 und 38 sind in dem gezeigten Ausführungsbeispiel vom proximalen Ende 52 bzw. 54 her auf die Griffteile 22 bzw. 24 aufschiebbar ausgebildet.

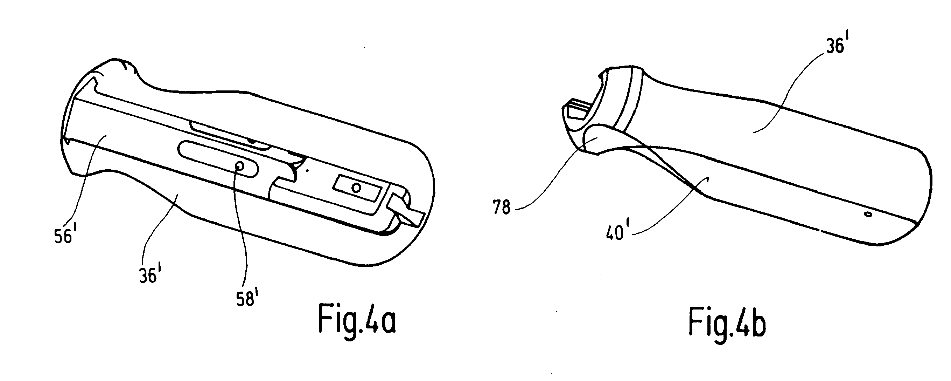

Die Griffschalen 36 und 38 weisen jeweils eine sich in Längsrichtung der Griffschalen 36 und 38 erstreckende Vertiefung oder Ausnehmung 56 auf, die in Form einer Nut ausgebildet ist, wobei die Ausnehmung oder Vertiefung 56 in Fig. 3 für die Griffschale 36 im Querschnitt dargestellt ist. Hierzu wird auch auf Fig. 4b) bzw. 5a) verwiesen, in denen Griffschalen 36' bzw. 38' dargestellt sind, die eine als Nut ausgebildete Vertiefung oder Ausnehmung 56' aufweisen, die der Vertiefung 56 der Griffschalen 36 und 38 entspricht.

In der Vertiefung oder Ausnehmung 56 sind die Griffteile 22 bzw. 24 im befestigten Zustand der Griffschalen 36 und 38 zumindest teilweise, im gezeigten Ausführungsbeispiel der Fig. 1 bis 3 im Querschnitt gesehen vollständig aufgenommen.

Die Griffschalen 36 und 38 weisen jeweils zumindest ein Element 58 und 60 zum Festlegen der Griffschalen 36 und 38 an den Griffteilen 22 und 24 auf.

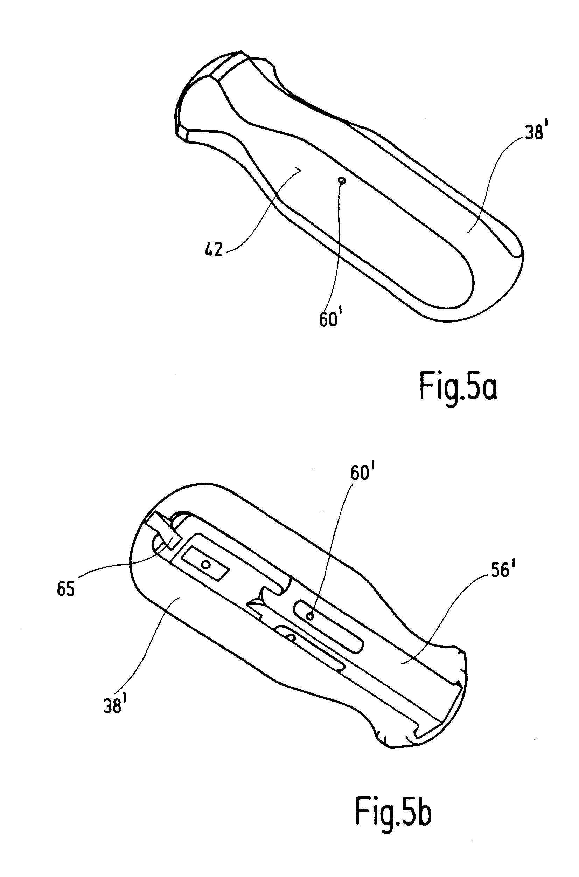

Die Elemente 58 und 60 sind beispielsweise als Schrauben, beispielsweise Madenschrauben 62, 64 ausgebildet, die durch entsprechende Öffnungen in den Griffschalen 36 bzw. 38 eingedreht und mit den Griffteilen 22 und 24 klemmend in Eingriff gebracht werden können. Zu den Griffteilen 22 und 24 könnten aber auch Gewinde- oder Zylinderlöcher vorhanden sein, in die die Schrauben 62, 64 eingedreht werden oder eingreifen können. Die Elemente 58 bzw. 60 legen damit die Griffschalen 36 bzw. 38 in Längsrichtung der Griffteile 22 bzw. 24 an diesen fest. Anstelle durch Verschraubung oder zusätzlich können die Griffschalen 36 und 38 jedoch auch beispielsweise durch eine Raste 65 (vgl. Fig. 5a) an den Griffteilen 22 bzw. 24 festgelegt werden.

Darüber hinaus wird eine Festlegung der Griffschalen 36 und 38 bei dem Ausführungsbeispiel gemäß Fig. 1 bis 3 auch dadurch realisiert, daß die Griffteile 22 und 24 in der jeweiligen Vertiefung oder Ausnehmung 56 der Griffschale bzw. 38 allseitig im wesentlichen eingefaßt sind, wie aus Fig. 3 hervorgeht. Auf diese Weise sind die Griffschalen 36 und 38 auch in Querrichtung zu den Griffteilen 22 und 24 an diesen festlegbar.

Gemäß der in dem Ausführungsbeispiel der Fig. 1 bis 3 im Querschnitt nicht runden, sondern eckigen Ausgestaltung der Griffteile 22 und 24 bewirkt die im wesentlichen formschlüssige Aufnahme der Griffteile 22 und 24 in den Griffschalen 36 bzw. 38 auch eine Verdrehsicherung der Griffschalen 36 und 38 um die Längsrichtung der Griffteile 22 bzw. 24.

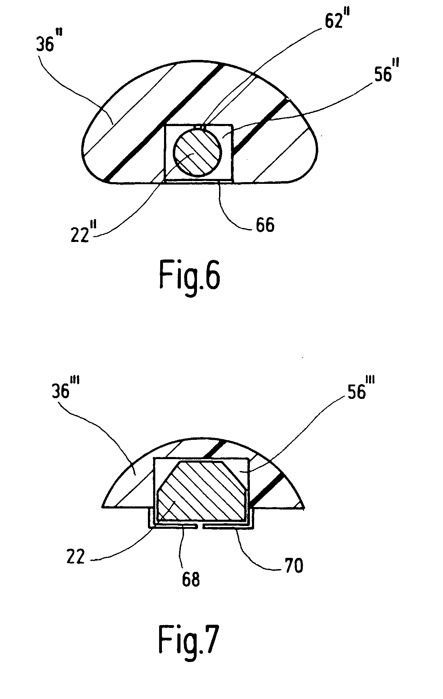

Im Falle einer zylindrischen Ausgestaltung eines Griffteils 22" können jedoch auch Schrauben eine derartige Verdrehsicherung bewirken, wie beispielsweise in Fig. 6 für ein Griffteil 22" mit rundem Querschnitt und einer Griffschale 36'' mit Schraube 62'' dargestellt ist, bei der das Griffteil 24' in der Ausnehmung oder Vertiefung 56'' der Griffschale 36'' nicht formschlüssig aufgenommen ist.

Um bei dem in Fig. 6 dargestellten Ausführungsbeispiel die Griffschale 36'' auch in Querrichtung, d.h. quer zur Längsrichtung des Griffteils 22'', insbesondere in Betätigungsrichtung des Griffteils 22'', festzulegen, weist die Griffschale 36'' zumindest eine Lasche 66 auf, die das Griffteil 22'' zumindest teilweise, gemäß Fig. 6 vollständig umgreift. Über die Länge der Griffschale 36'' können eine Mehrzahl derartiger Laschen 66 vorgesehen sein, oder die Vertiefung bzw. Ausnehmung 56'' kann als im Querschnitt allseitig geschlossene Bohrung oder Ausnehmung ausgeführt sein.

In Fig. 7 ist ein Ausführungsbeispiel dargestellt, wonach eine Griffschale 36''' als Klemmteil zum Anklemmen an das Griffteil 22 ausgebildet ist. In dieser Ausgestaltung kann die Schale 36''' in Längsrichtung des Griffteils 22 auf dieses aufgeschoben werden. Bei dem in Fig. 7 dargestellten Ausführungsbeispiel weist die Griffschale 36''' ferner eine Vertiefung oder Ausnehmung 56''' auf, in der das Griffteil 22 nur teilweise aufgenommen ist. Zum Anklemmen der Griffschale 36''' an dem Griffteil 22 weist die Griffschale 36''' Laschen 68 und 70 auf, die entsprechend elastisch ausgebildet sind und als Klemmen wirken. Die Laschen 68 und 70 dienen gleichzeitig als Elemente zum Festlegen der Griffschale 36''' in Querrichtung zu dem Griffteil 22, d.h. quer zur Längsrichtung, insbesondere in Betätigungsrichtung des Griffteils 22. Bei entsprechender Ausgestaltung der Laschen 68 und 70 als elastische Elemente und bei entsprechender Geometrie der Laschen 68 und 70 könnte die Griffschale 36''' auch quer zur Längsrichtung des Griffteils 22 in der Art eines Clips auf dieses aufgesteckt werden, wobei beim Aufstecken die Laschen 68 und 70 sich dann auseinanderspreizen und anschließend um das Griffteil 22 herumlegen bzw. schließen.

Wieder mit Bezug auf Fig. 2 und 3 weisen die Griffschalen 36 und 38 Verriegelungsmittel 72 zum Verriegeln der Griffteile 22 und 24 in verschiedenen relativen Griffstellungen zueinander auf. Die Verriegelungsmittel 72 weisen beispielsweise eine mit der Griffschale 36 verbundene Hülse 74 und einen mit der Griffschale 38 verbundenen Stift 76 auf, der unter Reibschluß in die Hülse 74 eingreift und eine stufenlose Verriegelung der Griffteile 22 und 24 aneinander ermöglicht.

Die in Fig. 4 und 5 dargestellten Griffteile 36' und 38' können ebenfalls bei dem Instrument 10 an den Griffteilen 22 und 24 befestigt werden, wobei das Griffteil 36' eine Grifffläche 40' aufweist, die insbesondere an die Handinnenfläche im Bereich des Daumens angepaßt ist und vorzugsweise eine Daumenmulde 78 aufweist, während die Griffschale 38' eine Grifffläche 42' aufweist, die an den unteren Bereich der Handinnenfläche angepaßt ist.

Die Griffflächen 40 und 42 der Griffschalen 36 und 38 sind vorzugsweise weich und/oder aufgerauht. Die Griffschalen 36 und 38 sind ferner autoklavierbar.

Es versteht sich, daß für das Instrument 10 mehrere Sätze von Griffschalen 36 und 38 unterschiedlicher Größe und/oder unterschiedlicher Formen bereitgehalten werden können, um die Handhabe 20 des Instruments 10 an die jeweiligen Bedürfnisse und Anforderungen der Bedienungsperson anpaßbar zu machen.

## Patentansprüche

1. Medizinisches Instrument (10), mit einer Handhabe (20), die zwei im wesentlichen stabförmige Griffteile (22, 24; 22'') aufweist, die jeweils eine Grifffläche (30, 32) aufweisen, und mit einer Griffanordnung (34), die für jedes Griffteil (22, 24; 22'') eine an dem jeweiligen Griffteil (22, 24; 22'') im Bereich deren Grifffläche (30, 32) abnehmbar befestigbare Griffschale (36, 38; 36', 38'; 36''; 36''') mit jeweils einer gegenüber der Grifffläche (30, 32) der Griffteile (22, 24; 22'') vergrößerten und/oder formveränderten Grifffläche (40, 42; 40', 42') aufweist dadurch charakterisiert, dass die Griffschalen (36, 38; 36', 38'; 36''; 36''') zum greifen des medirinischen Instruments vom proximalen Ende der Griffteile (22, 24; 22'') her auf die-se aufschiebbar ausgebildet sind.

2. Instrument nach Anspruch 1, wobei die Griffschalen (36, 38; 36', 38'; 36'';36''') seitlich auf die Griffteile (22, 24; 22'') aufsteckbar ausgebildet sind.

3. Instrument nach Anspruch 1 oder 2, wobei die Griffschalen (36''') als Klemmteile zum Anklemmen an die Griffteile (22, 24) ausgebildet sind.

4. Instrument nach einem der Ansprüche 1 bis 3, wobei die Griffschalen (36, 38; 36', 38'; 36''; 36''') eine Vertiefung oder Ausnehmung (56; 56'; 56''; 56''') aufweisen, in der die Griffteile (22, 24; 22'') im befestigten Zustand der Griffschalen (36, 38; 36', 38'; 36''; 36''') zumindest teilweise aufgenommen sind.

5. Instrument nach Anspruch 4, wobei die Vertiefungen oder Ausnehmungen (56; 56'; 56'') so ausgebildet sind, daß die Griffteile (22, 24; 22'') darin im Querschnitt gesehen vollständig aufgenommen sind.

6. Instrument nach einem der Ansprüche 1 bis 5, wobei im an den Griffteilen (22, 24; 22'') befestigten Zustand einander zugewandte Seiten (44, 46) der Griffschalen (36, 38; 36', 38'; 36'') randseitig jeweils abgerundet ausgebildet sind.

7. Instrument nach einem der Ansprüche 1 bis 6, wobei die Griffschalen (36, 38; 36', 38'; 36''; 36''') jeweils zumindest ein Element (58, 60) zum Festlegen der Griffschalen an den Griffteilen (22, 24; 22'') in Längsrichtung und/oder Querrichtung zu den Griffteilen (22, 24; 22'') und/oder zum Verdrehsichern der Griffschalen (36, 38; 36', 38'; 36"; 36''') um die Längsrichtung der Griffteile (22, 24; 22'') aufweisen.

8. Instrument nach Anspruch 7, wobei das zumindest eine Element (58, 60) eine Schraube (62, 62) oder eine Raste (65) ist.

9. Instrument nach Anspruch 7 oder 8, wobei das zumindest eine Element eine Lasche (66; 68, 70) ist, die das entsprechende Griffteil (22; 22'') zumindest teilweise umgreift.

10. Instrument nach Anspruch 4 oder einem der Ansprüche 5 bis 9, soweit diese auf Anspruch 4 rückbezogen sind, wobei die Ausnehmungen oder Vertiefungen (56; 56'; 56''') zur im wesentlichen formschlüssigen Aufnahme der Griffteile (22, 24) ausgebildet sind.

11. Instrument nach einem der Ansprüche 1 bis 10, wobei die Griffschalen (36, 38; 36', 38'; 36"; 36''') jeweils eine weiche und/oder aufgerauhte Grifffläche aufweisen.

12. Instrument nach einem der Ansprüche 1 bis 11, wobei die Griffschalen (36, 38) Verriegelungsmittel (72) zum Verriegeln der Griffteile (22, 24) in verschiedenen relativen Griffstellungen aufweisen, die vorzugsweise eine stufenlose Verriegelung ermöglichen.

13. Instrument nach einem der Ansprüche 1 bis 12, wobei es eine Mehrzahl von Sätzen von Griffschalen (36, 38; 36', 38'; 36''; 36''') unterschiedlicher Größen und/oder Formen aufweist.

14. Instrument nach einem der Ansprüche 1 bis 13, wobei die Griffschalen (36, 38; 36', 38'; 36'';36''') autoklavierbar sind.

## Claims

1. A medical instrument (10), comprising a handle (20) having two substantially rod-shaped grip parts (22, 24; 22") each having a grip surface (30, 32), and a grip arrangement (34) having, for each grip part (22, 24; 22"), a grip shell (36, 38; 36', 38'; 36"; 36''') which can be secured in a detachable manner on the respective grip part (22, 24; 22") in the region of the grip surface (30, 32) thereof, and which in each case has a grip surface (40, 42; 40', 42') which is of a larger size and/or of a different shape compared to the grip surface (30, 32) of the grip parts (22, 24; 22"), **characterized in that** the grip shells (36, 38; 36', 38'; 36"; 36'") are configured so that they can be pushed onto the grip parts (22, 24; 22") from the proximal end thereof for gripping the medical instrument.

2. The instrument of claim 1, wherein the grip shells (36, 38; 36', 38'; 36"; 36''') are configured so that they can be plugged onto the grip parts (22, 24; 22") from the side.

3. The instrument of claim 1 or 2, wherein the grip shells (36''') are configured as clamp parts to be clamped onto the grip parts (22, 24).

4. The instrument of any of claims 1 through 3, wherein the grip shells (36, 38; 36', 38'; 36"; 36'") have a depression or recess (56; 56'; 56"; 56''') in which the grip parts (22, 24; 22") are at least partially received when the grip shells (36, 38; 36', 38'; 36"; 36''') are secured.

5. The instrument of claim 4, wherein the depressions or recesses (56; 56'; 56") are configured so that the grip parts (22, 24; 22") are received completely therein, seen in cross section.

6. The instrument of any of claims 1 through 5, wherein, when secured on the grip parts (22, 24; 22"), mutually facing sides (44, 46) of the grip shells (36, 38; 36', 38'; 36"), are each rounded along their edge.

7. The instrument of any of claims 1 through 6, wherein the grip shells (36, 38; 36', 38'; 36"; 36''') each have at least one element (58, 60) for fixing the grip shells on the grip parts (22, 24; 22") in the longitudinal direction and/or transverse direction with respect to the grip parts (22, 24; 22") and/or for securing the grip shells (36, 38; 36', 38'; 36"; 36''') against rotating about the longitudinal direction of the grip parts (22, 24; 22'').

8. The instrument of claim 7, wherein the at least one element (58, 60) is a screw (62, 64) or a catch (65).

9. The instrument of claim 7 or 8, wherein the at least one element is a bracket (66; 68, 70) which engages at least partially around the corresponding grip part (22; 22'').

10. The instrument of claim 4 or any of claims 5 through 9, insofar as these refer back to claim 4, wherein the recesses or depressions (56; 56'; 56''') are configured to receive the grip parts (22, 24) substantially with a form fit.

11. The instrument of any of claims 1 through 10, wherein the grip shells (36, 38; 36', 38'; 36"; 36'") each have a soft and/or roughened grip surface.

12. The instrument of any of claims 1 through 11, wherein the grip shells (36, 38) have locking means (72) for locking the grip parts (22, 24) in different relative grip positions, and which preferably permit stepless locking.

13. The instrument of any of claims 1 through 12, wherein it comprises a plurality of sets of grip shells (36, 38; 36', 38'; 36"; 36''') of different sizes and/or shapes.

14. The instrument of any of claims 1 through 13, wherein the grip shells (36, 38; 36', 38'; 36"; 36''') can be autoclaved.

## Revendications

1. Instrument médical (10), comprenant une poignée (20), qui présente deux parties de préhension (22, 24 ; 22") présentant essentiellement une forme de barre, lesquelles présentent respectivement une surface de préhension (30, 32), et comprenant un ensemble de préhension (34), qui présente pour chaque partie de préhension (22, 24 ; 22") une coque de préhension (36, 38 ; 36', 38' ; 36" ; 36"') pouvant être fixée de manière détachable au niveau de la partie de préhension respective (22, 24 ; 22") dans la zone de la surface de préhension (30, 32) de cette dernière et dotée respectivement d'une surface de préhension (40, 42 ; 40', 42') à forme variable et/ou plus grande par rapport à la surface de préhension (30, 32) des parties de préhension (22, 24 ; 22"), **caractérisé en ce que** les coques de préhension (36, 38 ; 36', 38' ; 36" ; 36''') servant à saisir l'instrument médical sont réalisées de manière à pouvoir être enfilées sur les parties de préhension (22, 24 ; 22") depuis l'extrémité proximale de ces dernières.

2. Instrument selon la revendication 1, sachant que les coques de préhension (36, 38 ; 36', 38' ; 36" ; 36"') sont réalisées de manière à pouvoir être enfilées sur le côté des parties de préhension (22, 24 ; 22").

3. Instrument selon la revendication 1 ou 2, sachant que les coques de préhension (36"') sont réalisées sous la forme de parties de serrage destinées à être serrées sur les parties de préhension (22, 24).

4. Instrument selon l'une quelconque des revendications 1 à 3, sachant que les coques de préhension (36, 38 ; 36', 38' ; 36" ; 36"') présentent un renfoncement ou un évidement (56, 56' ; 56" ; 56"'), dans lequel les parties de préhension (22, 24 ; 22") sont logées au moins en partie lorsque les coques de préhension (36, 38 ; 36', 38' ; 36" ; 36"') se trouvent dans l'etat fixé.

5. Instrument selon la revendication 4, sachant que les renfoncements ou évidements (56 ; 56' ; 56") sont réalisés de telle manière que les parties de préhension (22, 24 ; 22") sont logées intégralement dans ces derniers vus dans la section transversale.

6. Instrument selon l'une quelconque des revendications 1 à 5, sachant que des côtés (44, 46) tournés les uns vers les autres des coques de préhension (36, 38 ; 36', 38' ; 36") sont réalisés, à l'état fixé au niveau des parties de préhension (22, 24 ; 22"), respectivement de manière arrondie côté bord.

7. Instrument selon l'une quelconque des revendications 1 à 6, sachant que les coques de préhension (36, 38 ; 36', 38' ; 36" ; 36'") présentent respectivement au moins un élément (58, 60) servant à fixer les coques de préhension au niveau des parties de préhension (22, 24 ; 22") dans la direction longitudinale et/ou dans la direction transversale par rapport aux parties de préhension (22, 24 ; 22") et/ou servant à empêcher les coques de préhension (36, 38 ; 36', 38' ; 36" ; 36"') de tourner autour de la direction longitudinale des parties de préhension (22, 24 ; 22").

8. Instrument selon la revendication 7, sachant que l'élément (58, 60) au moins au nombre de un est une vis (62, 62) ou un dispositif d'enclenchement (65).

9. Instrument selon la revendication 7 ou 8, sachant que l'élément au moins au nombre de un est une patte de fixation (66 ; 68, 70), qui saisit au moins en partie la partie de préhension (22 ; 22") correpondante.

10. Instrument selon la revendication 4 ou selon l'une quelconque des revendications 5 à 9, dans la mesure où ces dernières se rapportent à la revendication 4, sachant que les évidements ou renfoncements (56 ; 56' ; 56"') sont réalisés aux fins du logement essentiellement par complémentarité de forme des parties de préhension (22, 24).

11. Instrument selon l'une quelconque des revendications 1 à 10, sachant que les coques de préhension (36, 38 ; 36', 38' ; 36" ; 36"') présentent respectivement une surface de préhension non rigide et/ou rugueuse.

12. Instrument selon l'une quelconque des revendications 1 à 11, sachant que les coques de préhension (36, 38) présentent des moyens de verrouillage (72) servant à verrouiller les parties de préhension (22, 24) dans diverses positions de préhension relatives, lesquelles permettent de préférence un verrouillage continu.

13. Instrument selon l'une quelconque des revendications 1 à 12, sachant qu'il présente une pluralité de jeux de coques de préhension (36, 38 ; 36', 38' ; 36" ; 36'") de diverses tailles et/ou formes.

14. Instrument selon l'une quelconque des revendications 1 à 13, sachant que les coques de préhension (36, 38 ; 36', 38' ; 36" ; 36"') peuvent passer à l'autoclave.
